# EUROPEAN PATENT APPLICATION

(11) **EP 4 534 047 A1**
(43) Date of publication of application: **09.04.2025**
(21) Application number: 24802148.7
(22) Date of filing: 23.02.2024
(51) Int. Cl.: A61F 2/24

(54) **VALVE PROSTHESIS DEVICE HAVING SEGMENTED TAPERED STRUCTURE**

(30) Priority: 24.08.2023 CN 202311077193
(71) Applicant: Shanghai Quanxin Medical Technology Co., Ltd, Shanghai 201615 (CN)
(72) Inventor: YU, Peng, Shanghai 201615 (CN); SUN, Chao, Shanghai 201615 (CN); WEI, Pan, Shanghai 201615 (CN)
(74) Representative: Ipside
(86) International application number: PCT/CN2024/078243
(87) International publication number: WO 2025/039492

(57) **Abstract**

A prosthetic valve device with a segmented tapered structure provided by the present invention comprises an outer stent and an inner stent, wherein the outer stent comprises a discoid structure deployed on an atrial side, a annular structure adapted to an annulus side, and a split structure with different tapers deployed on a ventricular side; resilient clamping parts are distributed on a circumferential side of the outer stent depending on original valve leaflets; the inner stent is connected to the inside of the outer stent, and at least one valve body is provided in the inner stent. Barbs or protrusions selectively or globally distributed on the annular structure of the outer stent are also provided for anchoring a prosthesis to a natural cardiac valve annulus. The discoid structure and the annular structure are circular structures or special-shaped structures, and the split structures with different tapers are distributed at 60°-120°. The use of a tapered structure with a split body reduces outflow obstructions and improves fitness of prosthesis.

## Description

### Technical Field

The present invention relates to cardiac surgical instruments, in particular, to a prosthetic valve device with a segmented tapered structure.

### Background of the Invention

At present, the conditions affecting the function of the mitral valve include: mitral regurgitation, mitral leaflet prolapse and mitral stenosis. Mitral regurgitation refers to the leakage of blood flow from the left ventricle to the left atrium caused by the inability of the mitral leaflets to coapt at peak systolic blood pressure of the heart. Structural reasons that lead to the failure of normal closure of the mitral valve leaflets are: leaflet damage, annular enlargement, chordal rupture and papillary muscle ischemia. Leaflet prolapse or structural abnormality of the leaflet caused by the leaflet protrusion toward the atrial side may also contribute to mitral regurgitation. The normal function of mitral valve can also be compromised by mitral stenosis or narrowing of the mitral valve orifice, causing obstruction of diastolic blood flow from the atria through the ventricles.

Mitral regurgitation is mainly treated medically and surgically to reduce regurgitation into the atrium. For example, the dilated annulus is clamped or sheared in a repair manner to allow the leaflets to coapt well. The annulus is clamped by implanting a circumferential ring around the annulus to constrict the dilated annulus. Other repair procedures may involve suturing or clamping the leaflets to provide good leaflet apposition. Alternatively, more interventional procedures include replacement of the entire natural valve with a mechanical or bioprosthetic valve. This procedure is performed more often by extracorporeal circulation through a thoracotomy, which can make the patient more invasive, more likely to cause pain and death and require a longer recovery time.

A less invasive aortic valve replacement has been performed in recent years, with the aortic prosthetic valves including expandable stents as well as a trileaflet valve. Expandable stents are designed to fit into the relatively rigid aortic root of a symmetrical circumferential pattern, and due to these characteristics of the aortic root, the expandable stents can fit into the aortic root and is not easily displaced or shed.

Compared with the anatomy of aortic valves, the complex anatomy of the mitral valve poses a challenge for the mitral valve replacement. First, unlike relatively symmetrical and natural rigid aortic annuli, mitral annuli are saddle-shaped, lack symmetry, and vary greatly in size during the cardiac cycle. These uncertainties pose challenges in designing an appropriate prosthetic valve that can fit into the mitral annulus. Insufficient contacts between the prosthetic valve and the mitral annulus, resulting in the presence of a gap, will lead to regurgitation of blood flow. However, a paravalvular leakage may result if an expandable stent is implanted.

Current aortic valve replacement devices are not designed to fit the mitral valve. First, many devices require a straightforward configuration to fit the mitral annulus as well as to attach a support leaflet prosthesis. For some devices, the stent structures used to support the valve leaflets also fit the annulus or other surrounding tissues, and are subject to deformation pressures directly from the annulus or surrounding tissues or blood during the systole. Most prosthetic valves use trileaflet valves, and this design requires symmetrical circumferential support structures to allow the trileaflet valves to open and close freely for prolonged periods. If this support structure is subjected to bending deformation by deformation pressures from the annulus or surrounding tissue, the trileaflet valves may fail functionally.

In addition to the irregular and uncertain shape, the mitral annulus lacks important radial support structures. The aortic valve is surrounded by fibroelastic tissues at the root to aid in anchoring the prosthetic valve. The mitral valve is simply encased in cardiac tissues, thus, the mitral annulus possesses a strong radial force that may cause a structural failure of the implanted prosthetic valve.

Chordal tissues in the ventricles may also affect the prosthetic valve. This chordal aortic valve is absent. This chordae creates confusion for the interventional repair or replacement of the mitral valve through a sheath, increasing the difficulty of surgical positioning and placement, while positioning or placement of prosthetic valves from ventricular sides can also be difficult due to chordal interference.

Although there are normally three leaflets in the tricuspid valve on the right side of the heart, it is no less difficult to treat than the mitral valve. Therefore, the treatment of tricuspid valves similarly requires a well-designed prosthetic valve.

Existing prosthetic valve devices allow essentially simple and efficient intervention to treat valvular heart diseases. In current technology, the deployment configuration on the ventricular side of the prosthetic valve devices is generally an integral tapered or annular structure. However this design makes the prosthetic device overall too high, occupying the ventricular side unnecessarily, which may cause obstructions of flow.

### Summary of the Invention

The present invention discloses a prosthetic valve device which adopts a segmented tapered structure on the ventricular side to reduce outflow obstructions and improve fitness of prostheses, rather than fitting the ventricular wall.

The present invention specifically adopts the following technical means:
A prosthetic valve device with a segmented tapered structure comprises an outer stent and an inner stent, wherein the outer stent comprises a discoid structure deployed on an atrial side, a annular structure adapted to an annulus side, and a split structure with different tapers deployed on a ventricular side; resilient clamping parts are distributed on a circumferential side of the outer stent depending on original valve leaflets; the inner stent is connected to the inside of the outer stent, and at least one valve body is provided in the inner stent.

Furtherly, the prosthetic valve device with a segmented tapered structure further comprises barbs or protrusions selectively or globally distributed on the annular structure of the outer stent for anchoring a prosthesis to a natural cardiac valve annulus.

Furtherly, the barbs or protrusions are distributed midway between leaflets of a tricuspid valve; or at a position of an anterior leaflet A2 and posterior leaflets P1 and P3 of a mitral valve.

Furtherly, the discoid structure deployed on the atrial side is a circular structure or a special-shaped structure and the annular structure deployed on the annular side is a circular structure or a special-shaped structure.

Furtherly, the special-shaped structure is a D-like or saddle-shaped structure.

Furtherly, the segmented structure with different tapers is distributed at 60°-120°, with the split structure of the prosthetic valve device adapted for the mitral valve distributed at 120°.

Furtherly, a developing structure that can be identified by ultrasound is provided on the discoid structure of the atrial side.

Furtherly, the inner stent and the outer stent are made of memory alloys or biocompatible metal materials, and the inner stent can be deployable or self-expanding.

Furtherly, the outer stent is cut and shaped from a tubular material made of memory alloys or woven and shaped from a filamentary material, and the inner stent is cut and shaped from a tubular material made of memory alloys.

Furtherly, the inner stent and outer stent are integrally manufactured or connected and sewn together.

Furtherly, the outer stent and inner stent are covered with biocompatible materials to avoid leakages of blood flow between a prosthesis and a natural annulus. The structures on the atrial, natural annulus, and ventricular sides are coated with PET, and the inner edge of the inner stent is coated with PET or other biocompatible material.

Furtherly, the clamping part comprises the annular structure woven from a filamentary material or cut and shaped from a flat plate.

Furtherly, the clamping part is affixed with a woven flexible material.

Furtherly, the prosthetic valve device adapted for use with the mitral valve comprises two clamping parts, with a front anchoring part used to capture a natural anterior leaflet and fix a prosthesis, and a rear anchoring part used to capture a natural posterior leaflet and fix a prosthesis; and wherein the prosthetic valve device adapted for use with a tricuspid valve comprises three clamping parts, with one of the clamping parts used to capture a natural anterior leaflet and fix a prosthesis, a rear clamping part used to capture a natural posterior leaflet and fix a prosthesis, and another clamping part used to capture a natural septal leaflet and fix a prosthesis.

Furtherly, the valve body is a biological tissue leaflet comprising either bovine pericardium or porcine pericardium.

The present invention has the following beneficial effects:
The selective distribution of barbs or protrusions to specific locations and reduction of nonessential anchoring structures can maintain the anchoring effect and minimize damages to the natural annulus, decreasing the impact on the conduction system such as the atrioventricular bundle and atrioventricular node, and decreasing the weight of the prosthetic valve device. The inner stent and outer stent are made of memory alloys, possessing excellent elasticity and mechanical properties. The annular structure of the outer stent can change with the valve annulus during the cardiac cycle, resulting in a good fit to the atrial wall above the valve annulus. The inner stent can provide support for the prosthetic valve device to prevent from collapsing. Clamping parts can be controlled by manipulating the handle to refold and rebound, and if the effect of capture is poor, the clamping part can be re-pulled for recapturing, increasing the success rate of procedures. The prosthetic valve stent combining the atrial, annular, and ventricular portions anchored at the natural valve device prevents the prosthetic valve from shifting or separating during the systole/diastole. The synergistic effect of all three is superior to the approach of having only one or a combination of only some of these anchoring modalities for the prosthetic valve.

### Brief Description of the Drawings

Figure 1 is a schematic diagram of the structure of the present invention;
Figure 2 is a schematic diagram of the structure of the present invention from another perspective;
Figure 3 is a schematic diagram of an outer stent according to an embodiment of the present invention;
Figure 4 is a schematic diagram of the outer stent from another perspective according to an embodiment of the present invention;
Figure 5 is a schematic diagram of the structure according to an embodiment of the present invention;
Figure 6 is a schematic diagram of the structure from another perspective according to an embodiment of the present invention;
Figure 7 is a schematic diagram of the structure according to another embodiment of the present invention;
Figure 8 is a schematic diagram of the structure according to another embodiment of the present invention;
Figure 9 is a schematic diagram of the distribution of barbs or protrusions according to another embodiment of the present invention;
Figure 10 is a schematic diagram of the structure with a developing structure of the present invention;

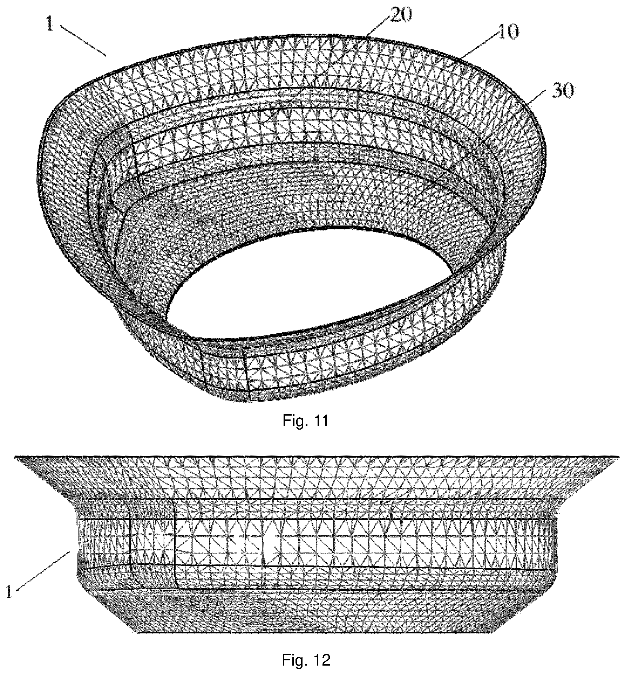

Figure 11 is a perspective view of an outer stent structure according to another embodiment of the present invention;
Figure 12 is a side view of an outer stent structure according to another embodiment of the present invention;
Figure 13 is a schematic diagram of the structure according to an embodiment of the present invention;
Figure 14 is a schematic diagram of the structure of the barbs of the present invention;
Figure 15 is a schematic diagram of the structure of the clamping part of the present invention;
Figure 16 is a schematic diagram of the structure of another clamping part of the present invention;
Figure 17 is a schematic diagram of the structure of an embodiment of an outer stent of the present invention;
Figure 18 is a schematic diagram of the structure of certain embodiments of the inner stent of the present invention; wherein Figures (m) and (n) respectively represent inner stents of two different structures;
Figure 19 is a schematic diagram of the structure of a valve body according to an embodiment of the present invention;
Figure 20 is a schematic diagram of the structure of the present invention in a folded state;

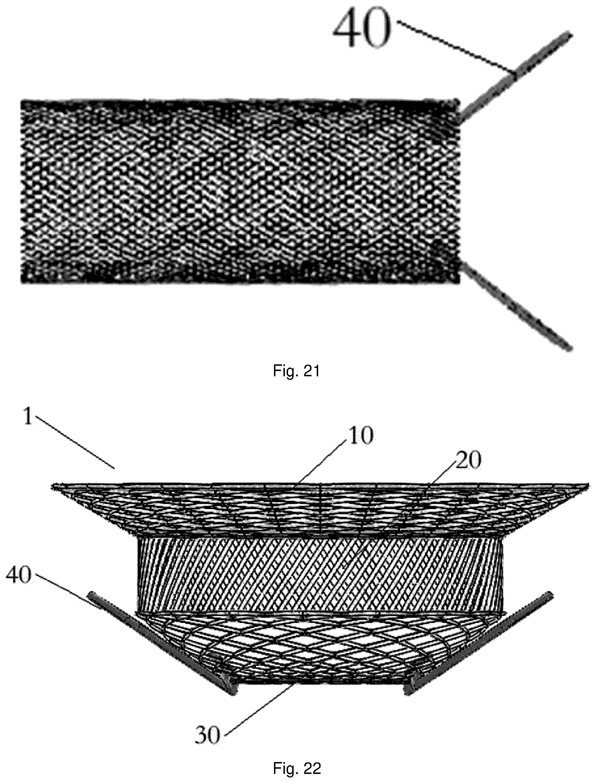

Figure 21 is a schematic diagram of the structure of the present invention in another folded state;
Figure 22 is a side view of the structure of the present invention in a deployed state;
Figure 23 is a perspective view of the structure of the present invention in a deployed state;
Figure 24 is a schematic diagram of the deployment process of the present invention through a sheath; wherein Figures (a)-(j) show the different assumed shape variations of the device during the deployment process;
Figure 25 is a schematic diagram of the structure of a mitral valve;
Figure 26 is a schematic diagram of transapical delivery;
Figure 27 is a schematic drawing of the deployment of the invention at completion.

The markups in the drawings are indicates as follows:
1-outer stent;
2-inner stent;
10-discoid structure;
20-annular structure;
30-split structure;
40-clamping part;
50-barb;
60-valve body;
70-coating;
80-sheath;
90-pull wire;
100-developing structure.

### Detailed Description of Embodiments

The objects, technical solutions and various advantages of the present invention will become apparent to one skilled in the art by reading the following specification with reference to the following drawings. Obviously, the described embodiments are merely exemplary in nature and are not representative of the entirety of the embodiments. Based on the embodiments of the present invention, all other embodiments obtained by those skilled in the art without making any creative work will fall within the scope of protection of the present invention.

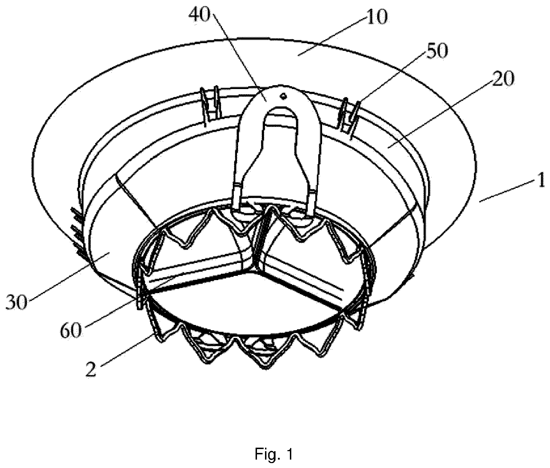

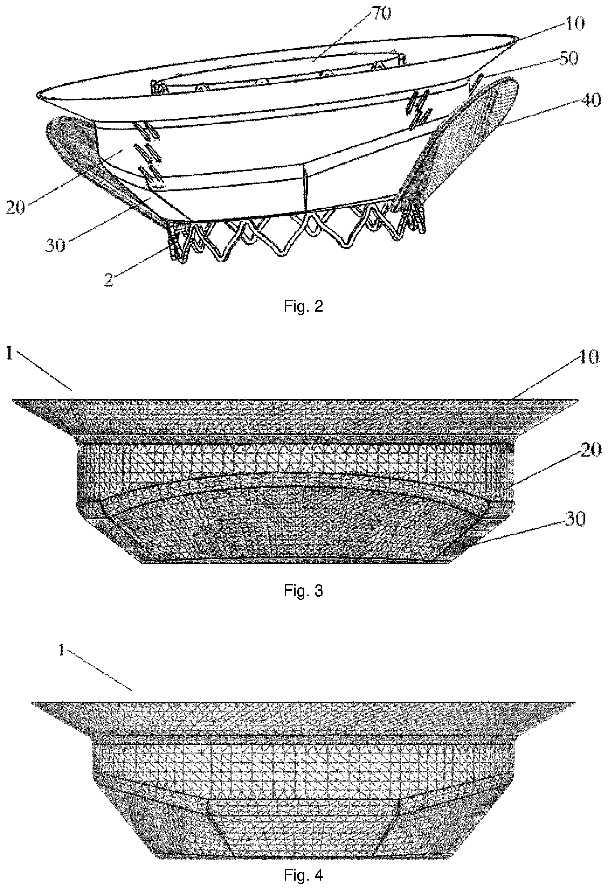

Referring to Figures 1 and 2, a prosthetic valve device comprises an outer stent 1 and an inner stent 2; wherein the outer stent 1 comprises a discoid structure 10 that deploys on the atrial side, an annular structure 20 that is adapted to fit the annulus on the annular side, and a split structure 30 with different tapers that deploys on the ventricular side; resilient clamping parts 40 are distributed on a circumferential side of the outer stent 1 depending on original valve leaflets; the inner stent 2 is connected to the inside of the outer stent 1, and at least one valve body is provided in the inner stent 2.

When deployed, the atrial side portion of the prosthetic valve is deployed radially to fit against the atrial wall and anchors at least one side of the prosthetic valve. The atrial side portion of the prosthetic valve has an axial low profile (extending only a short length toward the atrium) that minimizes thrombosis in the flow vortex. In a preferred embodiment, the atrial side portion of the prosthetic valve is covered with a biocompatible material such as PET or other synthetic prosthetic materials to seal the prosthetic valve on the atrial side, avoiding leakages between the prosthetic valve and the atrial wall when blood flows through.

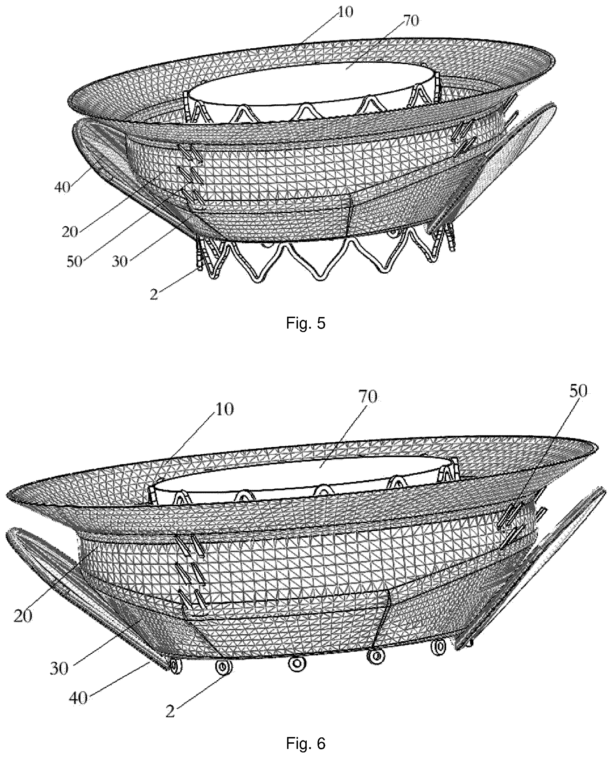

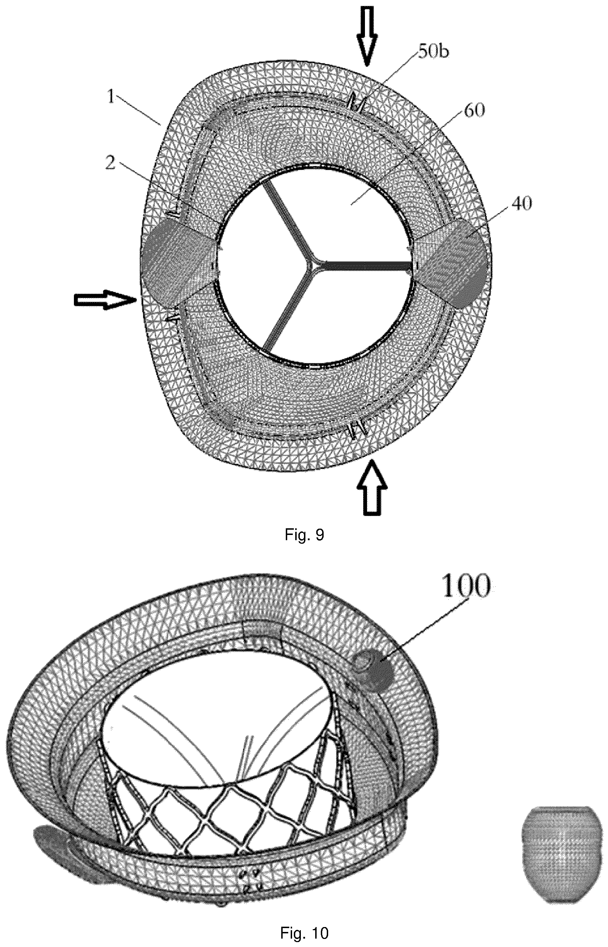

Referring to Figures 5-8, in some embodiments, the discoid structure 10 of the atrial side portion is circular. In some embodiments, the discoid structure 10 of the atrial side portion is a special-shaped structure, preferably a D-shaped structure or a saddle-shaped structure, which fits well into the atrial wall on the annulus. With reference to Figure 10, in order to enable the prosthetic valve to be positioned in the direction of deployment and release, in some embodiments, the discoid structure 10 of the atrial side portion is further provided with a development structure 100 that is particularly capable of being recognized by ultrasound, reducing the use of radiation development equipment.

The annular portion of the prosthetic valve is intended to be anchored to the natural annulus of the heart. In some embodiments, the annular structure 20 of the annular portion is circular. In some embodiments, the annular structure 20 of the annular portion is a special-shaped structure, preferably a D-shaped or saddle-shaped structure, which fits well into the atrial wall on the annulus.

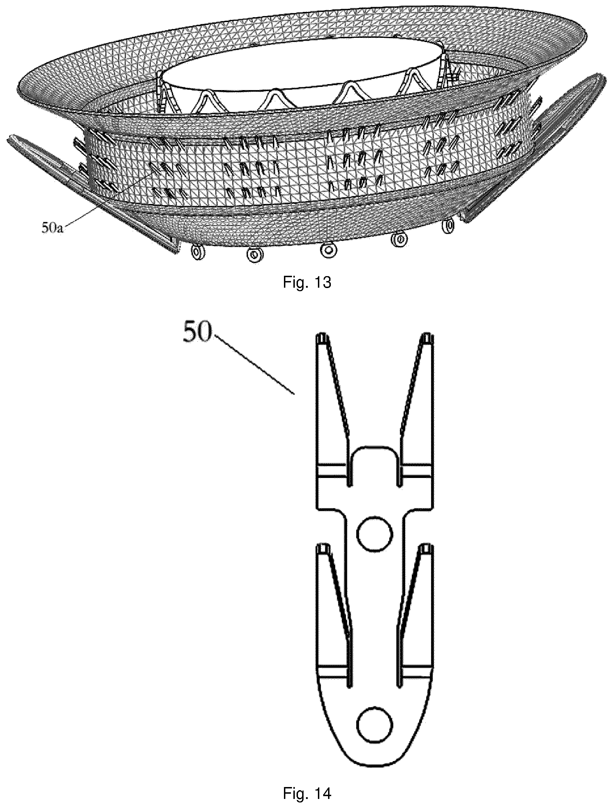

The annular portion of the prosthetic valve is distributed with barbs 50 or protrusion structures as in Figure 14, allowing the prosthetic device to better fit and anchor to the natural valve annulus. The barbs 50 cover the outer edge of the annulus of the outer stent 1 integrally, or are selectively distributed around the periphery of the prosthetic annulus depending on the characteristics of the natural valve, reducing damages to the natural annulus from excessive barbs or protrusions. Referring to Figure 13, the barbs 50a are equally distributed throughout. With reference to Figure 9 and the schematic of the mitral valve, when barbs 50b are locally distributed, the barbs or protrusions are distributed midway between leaflets of a tricuspid valve; or at a position of an anterior leaflet A2 and posterior leaflets P1 and P3 of a mitral valve. In a preferred embodiment, the valve annulus is covered with a biocompatible material, such as PET or other synthetic prosthetic materials, to seal the annulus portion for achieving no leakage between the prosthesis and the natural annulus when blood flows through.

When deployed, the ventricular side portion of the prosthesis is deployed radially and has an axial low profile with a split structure 30 of the same or different tapers, which reduces outflow obstructions or makes it more adaptable rather than apposing to the ventricular wall. In some embodiments, the split structure 30 with a taper on the ventricular side may exhibit a 60 °-120 ° distribution. For mitral valves, it is preferred that the distribution is at 120° to enable better adaptation. For tricuspid valves, preferably, the same taper split is used to enable better adaptation. In a preferred embodiment, the ventricular side portion of the prosthetic valve is covered with a biocompatible material, such as PET or other synthetic prosthetic materials, to seal the ventricular side portion for achieving no leakage between the prosthesis and the natural annulus when blood flows through.

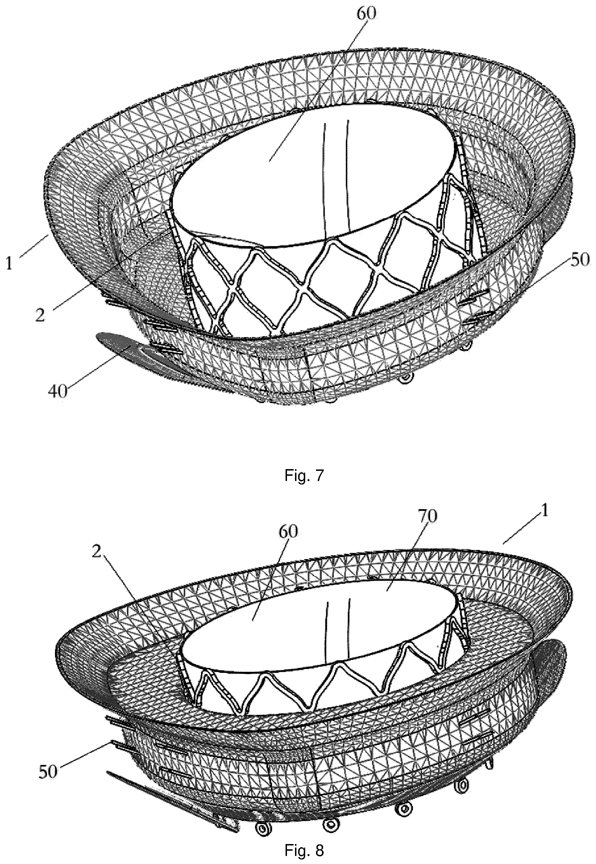

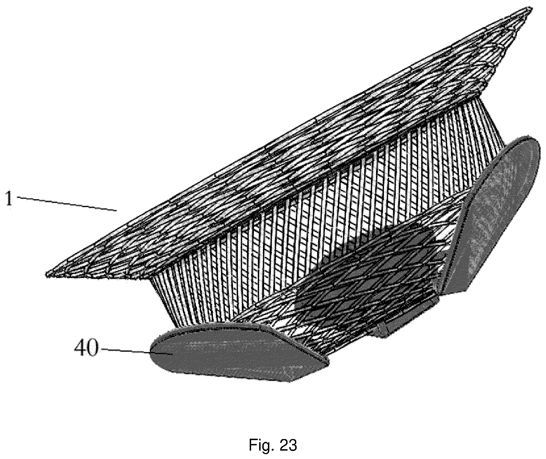

To anchor the prosthetic valve, clamping parts 40 are provided in the ventricular side portion, which are equally distributed around the circumference of the prosthetic valve depending on the characteristics of the natural leaflet. For the mitral valve, as shown in Figure 8, two clamping parts 40 are preferably used, with the anterior clamping part 40 used to capture the natural anterior leaflet and fix the prosthesis, and the posterior clamping part 40 used to capture the natural posterior leaflet and fix the prosthesis. For tricuspid valves, as shown in Figure 23, three clamping parts 40 are preferably used, with one of the clamping parts used to capture a natural anterior leaflet and fix the prosthesis, a rear clamping part used to capture a natural posterior leaflet and fix the prosthesis, and another clamping part used to capture a natural septal leaflet and fix the prosthesis.

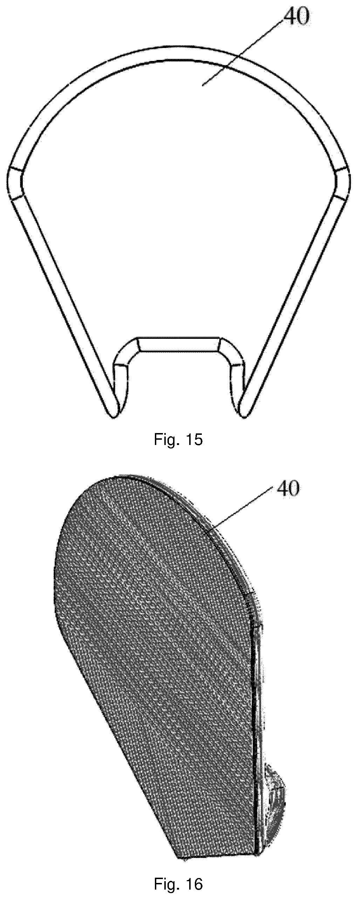

In some embodiments, the clamping part 40 as in Figure 15 comprises the annular structure woven from a filamentary material or cut and shaped from a flat plate, which can be stretched and rebounded to capture the natural leaflets between the outer stent 1 of the prosthetic valve.

The prosthetic valve stent, combined with the atrial, annular, and ventricular portions, anchored at the natural valve device prevents the prosthetic valve from shifting or separating during the systole/diastole. The synergistic effect of all three is superior to the approach of having only one or a combination of only some of these anchoring modalities for the prosthetic valve. In some embodiments, as shown in Figure 8, there is a certain gap between the structure of the prosthetic valve combined with the outer stent 1 and the inner stent 2 and the blood inflow side. When the outer stent 1 is extruded by the natural annulus on the inflow side, the outer stent 1 deforms towards the inner annulus in order to be able to fit with the natural annulus throughout the cardiac cycle, whereas the inner stent 2 will not be deformed, allowing for excellent hemodynamic performance of the prosthetic valve leaflets.

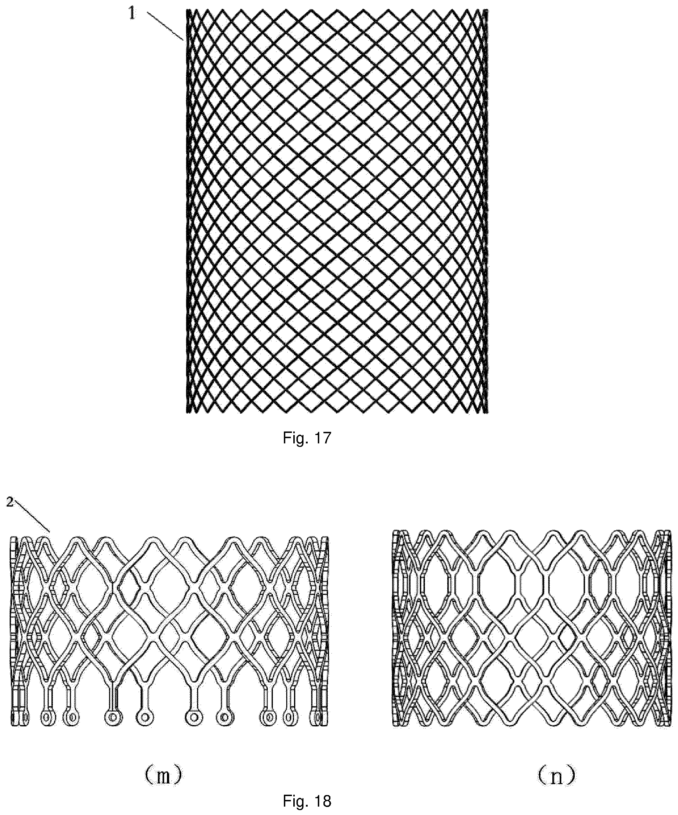

Referring to Figures 17-18, the inner stent 2 is designed to be deployable or capable of self-expanding. The inner stent 2 may be manufactured from a memory alloy, such as a nickel-titanium alloy, or other biocompatible metal materials. The outer stent 2 may also be manufactured from a memory alloy, such as a nickel-titanium alloy, or other biocompatible metal materials. The inner stent and outer stent can be integrally manufactured or connected and sewn together, the latter being used in this embodiment. In some embodiments, the annular structure 20 of the outer stent 1 may vary with the annulus during the cardiac cycle, designed as a D-shaped structure or a saddle-shape structure to conform to the annulus, especially for mitral valves. In some embodiments, the prosthetic valve device is cut and shaped from a tubular material made of memory alloys or woven and shaped from a filamentary material with good elasticity.

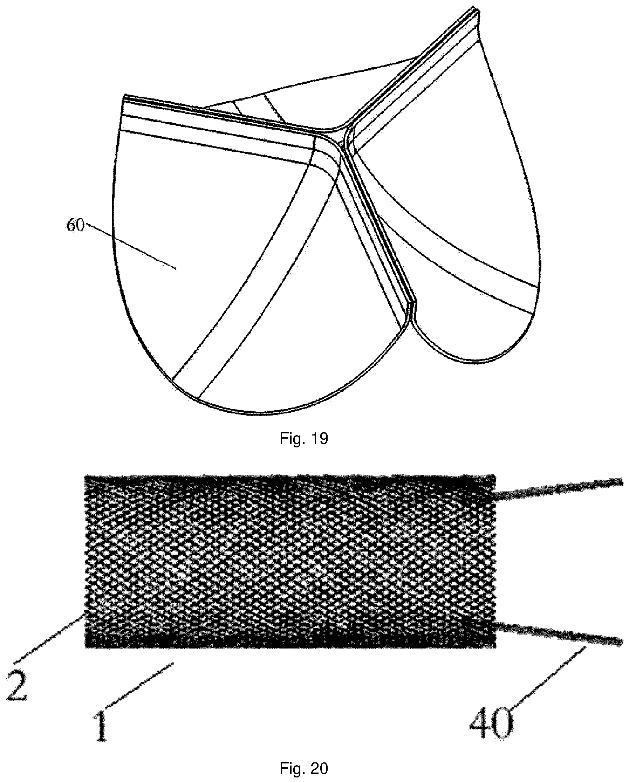

The inner stent 2 and outer stent 1 comprise a plurality of rhombic lattice units, with the valve body 60 sewn within the inner stent 2. In this embodiment, the valve body is a biological tissue leaflet and the biological valve tissue utilizes bovine pericardium or porcine pericardium as shown in Figure 19, comprising three separate valves or three valves molded in one piece.

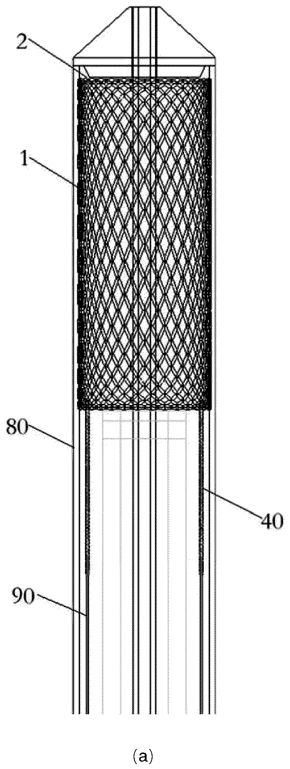

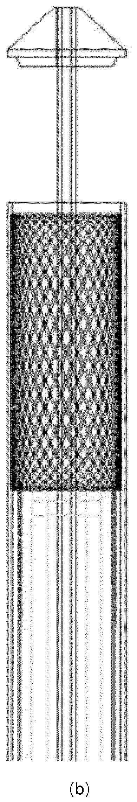

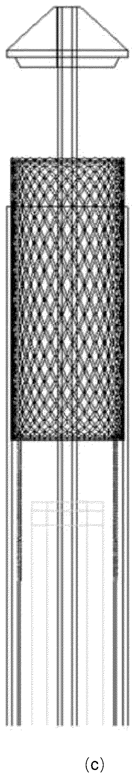

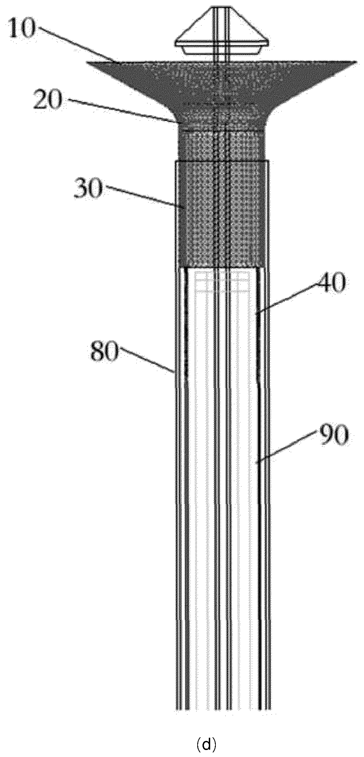

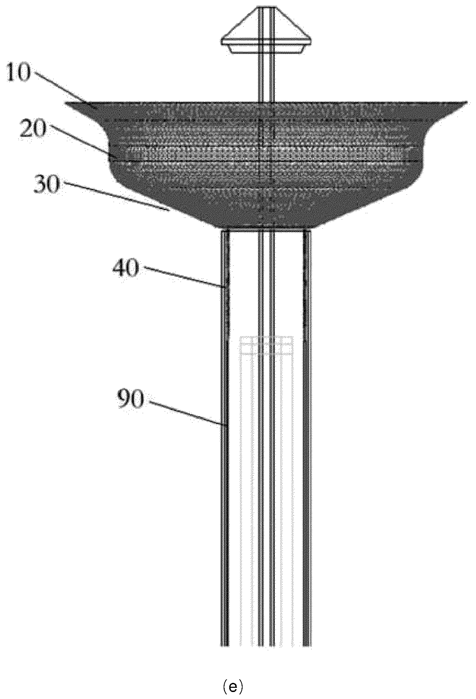

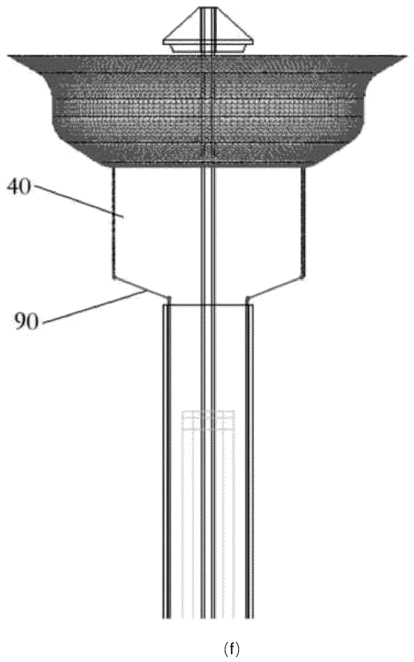

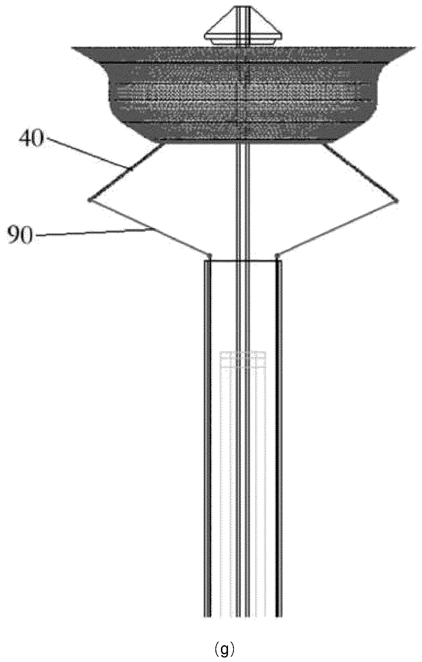

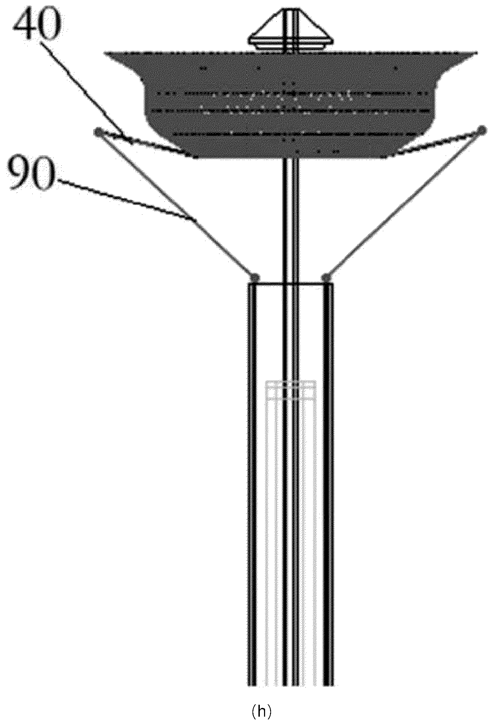

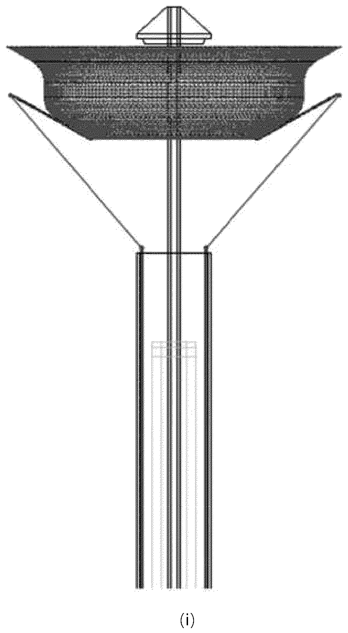

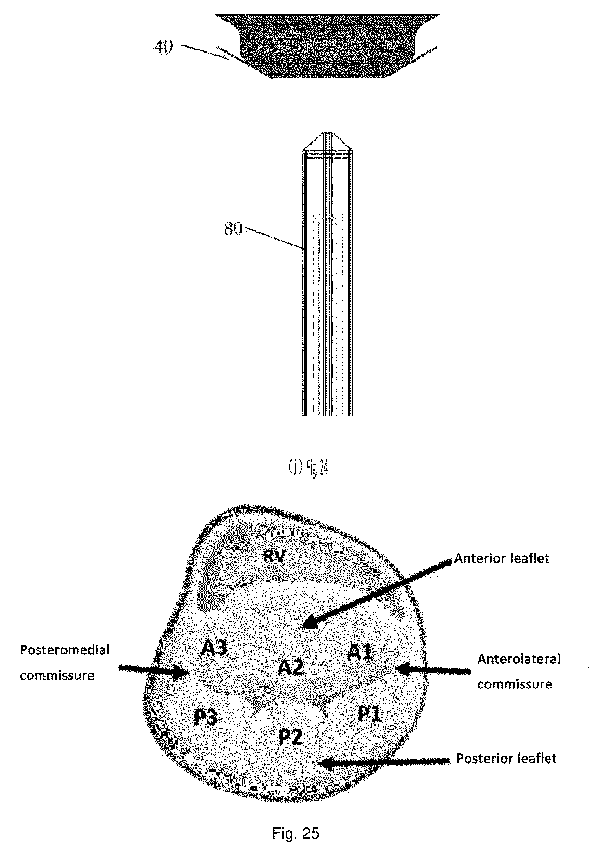

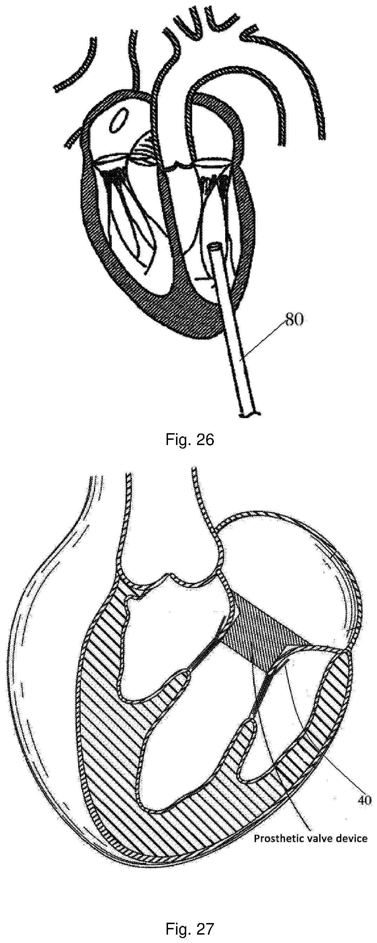

With reference to Figure 24, in order to release the prosthetic valve in the heart, the prosthetic valve is first compressed and loaded in a suitable sheath 80. The prosthetic valve is compressed into the sheath 80 and, based on the number of anchoring features of the prosthetic valve, the clamp part 40 is pulled back into the sheath 80 via a pull wire 90 or other similar wires. In some embodiments, the distal end of these pull wires 90 or other similar wires are connected to the prosthetic valve by a loop, and the rebound of the clamping part 40 is controlled by a manipulation handle at the proximal end of the pull wires 90 or other similar wires. As shown in Figure 26, the prosthetic valve loaded into sheath 80 is then subjected to transapical delivery. When the prosthetic valve is delivered to the designated atrial side, the sheath 80 is retracted by a delivery handle to gradually expose and deploy the prosthetic valve. In some embodiments with self-expanding stents, the prosthetic valve partially deploys once the prosthetic valve is progressively advanced for exposure, whereas in some embodiments with expandable stents, the assistance of a balloon is required for expansion and deployment.

Referring to Figures (a)-(j) for the prosthetic valve intervention process, the deployed prosthetic valve may be released according to the characteristics of the natural valve. In the embodiment of the mitral valve prosthesis, for example, the prosthetic valve comprises two clamping parts 40, located anteriorly and posteriorly. When a portion of the sheath 80 is withdrawn and the prosthetic valve is progressively pushed out, the atrial side portion of the prosthetic valve is already partially deployed, and the position of the natural leaflet relative to the prosthetic valve can be recognized with the guidance of ultrasound and can be adjusted to allow the anterior and posterior clamping parts 40 to fit with the natural leaflet. When the positions match, the sheath 80 is withdrawn again to allow the prosthetic valve to be gradually exposed, leading the atrial side portion and the annular portion of the prosthetic valve to unfold and fit into the natural annular device until the clamping parts 40 are exposed outside of the sheath, and then the withdrawal of the sheath is stopped. At this point, the prosthetic valve is well anchored at the natural annulus. The clamping parts 40 are then controlled to capture the natural leaflet by either a pull wire 90 or other mental wires, which subsequently allows the clamping parts 40 to be released for rebound. If the effect of capture is poor, the clamping parts 40 can be re-pulled for recapturing. Once the prosthetic valve is released in the desired position as well as the clamping part 40 is smoothly matched, the prosthetic valve is finally released after dissociating the pull wire or mental wires, prompting the two clamping parts 40 to capture the natural anterior and posterior leaflets between the prosthetic valve stent and the anchoring part, with the barbs 50 or protrusion structures supported under the natural annulus, the clamping parts 40 supported on the fibrous structure of the leaflets, and the prosthetic valve device anchored and stabilized. Finally, the sheath 80 is completely withdrawn and the implantation procedure is completed.

While preferred embodiments have been shown and described above, it will be apparent to those skilled in the art that changes and modifications could be made once the underlying inventive concepts as defined by the appended claims are learned. Therefore, the appended claims are intended to be construed to include the preferred embodiments as well as all changes and modifications that fall within the scope of the present invention.

It will be apparent to those skilled in the art that various modifications and variations can be made thereto without departing from the spirit and scope of the invention. All modifications and variations that fall within the bounds of the claims and equivalents are intended to be embraced.

## Claims

1. A prosthetic valve device with a segmented tapered structure, comprising an outer stent and an inner stent, wherein the outer stent comprises a discoid structure deployed on an atrial side, a annular structure adapted to an annulus side, and a split structure with different tapers deployed on a ventricular side; resilient clamping parts are distributed on a circumferential side of the outer stent depending on original valve leaflets; the inner stent is connected to the inside of the outer stent, and at least one valve body is provided in the inner stent.

2. The prosthetic valve device with a segmented tapered structure according to claim 1, further comprising barbs or protrusions selectively or globally distributed on the annular structure of the outer stent for anchoring a prosthesis to a natural cardiac valve annulus.

3. The prosthetic valve device with a segmented tapered structure according to claim 2, wherein the barbs or protrusions are distributed midway between leaflets of a tricuspid valve; or
at a position of an anterior leaflet A2 and posterior leaflets P1 and P3 of a mitral valve.

4. The prosthetic valve device with a segmented tapered structure according to claim 1, wherein the discoid structure deployed on the atrial side is a circular structure or a special-shaped structure and the annular structure deployed on the annular side is a circular structure or a special-shaped structure.

5. The prosthetic valve device with a segmented tapered structure according to claim 4, wherein the special-shaped structure is a D-like or saddle-shaped structure.

6. The prosthetic valve device with a segmented tapered structure according to claim 1, wherein the split structure with different tapers is distributed at 60°-120°, with the split structure of the prosthetic valve device adapted for the mitral valve distributed at 120°.

7. The prosthetic valve device with a segmented tapered structure according to claim 1, wherein a developing structure that can be identified by ultrasound is provided on the discoid structure of the atrial side.

8. The prosthetic valve device with a segmented tapered structure according to claim 1, wherein the inner stent and the outer stent are made of memory alloys or biocompatible metal materials, and the inner stent can be deployable or self-expanding.

9. The prosthetic valve device with a segmented tapered structure according to claim 1, wherein the outer stent is cut and shaped from a tubular material made of memory alloys or woven and shaped from a filamentary material, and the inner stent is cut and shaped from a tubular material made of memory alloys.

10. The prosthetic valve device with a segmented tapered structure according to claim 1, wherein the inner stent and outer stent are integrally manufactured or connected and sewn together.

11. The prosthetic valve device with a segmented tapered structure according to claim 1, wherein the outer stent and inner stent are covered with biocompatible materials to avoid leakages of blood flow between a prosthesis and a natural annulus.

12. The prosthetic valve device with a segmented tapered structure according to claim 1, wherein the clamping part comprises the annular structure woven from a filamentary material or cut and shaped from a flat plate.

13. The prosthetic valve device with a segmented tapered structure according to claim 12, wherein the clamping part is affixed with a woven flexible material.

14. The prosthetic valve device with a segmented tapered structure according to claim 1, wherein the prosthetic valve device adapted for use with the mitral valve comprises two clamping parts, with a front anchoring part used to capture a natural anterior leaflet and fix a prosthesis, and a rear anchoring part used to capture a natural posterior leaflet and fix a prosthesis; and wherein the prosthetic valve device adapted for use with a tricuspid valve comprises three clamping parts, with one of the clamping parts used to capture a natural anterior leaflet and fix a prosthesis, a rear clamping part used to capture a natural posterior leaflet and fix a prosthesis, and another clamping part used to capture a natural septal leaflet and fix a prosthesis.

15. The prosthetic valve device with a segmented tapered structure according to claim 1, wherein the valve body is a biological tissue leaflet utilizing either bovine pericardium or porcine pericardium.
